# EUROPÄISCHE PATENTSCHRIFT

(11) **EP 3 297 559 B1**
(45) Veröffentlichungstag und Bekanntmachung des Hinweises auf die Patenterteilung: **02.10.2019**
(21) Anmeldenummer: 16722239.7
(22) Anmeldetag: 13.05.2016
(51) Int. Cl.: A61B 18/14, A61B 18/00, A61B 17/00

(54) **ELEKTROCHIRURGISCHES KOAGULATIONSINSTRUMENT**
ELECTROSURGICAL COAGULATION INSTRUMENT
INSTRUMENT DE COAGULATION ÉLECTROCHIRURGICAL

(30) Priorität: 21.05.2015 DE 102015108078
(43) Veröffentlichungstag der Anmeldung: 28.03.2018
(73) Patentinhaber: Aesculap AG, 78532 Tuttlingen (DE)
(72) Erfinder: WEISSHAUPT, Dieter, 78194 Immendingen (DE); ROTHWEILER, Christoph, 78166 Donaueschingen (DE)
(74) Vertreter: Winter, Brandl, Fürniss, Hübner, Röss, Kaiser, Polte - Partnerschaft mbB
(86) Internationale Anmeldenummer: PCT/EP2016/060813
(87) Internationale Veröffentlichungsnummer: WO 2016/184796

(56) Entgegenhaltungen:
- EP-A1- 2 491 880
- DE-U1-202010 013 150
- US-A1- 2003 216 733
- US-A1- 2004 116 979
- US-A1- 2010 100 122

## Beschreibung

Die vorliegende Erfindung betrifft ein elektrochirurgisches Instrument zur Elektrokoagulation von Gewebe, mit einem Maulteil, welches zwei Instrumentenbranchen oder Klemmbacken aufweist, von denen zumindest eine gegenüber der anderen bewegbar ist, so dass das Maulteil in eine geöffnete Stellung oder in eine geschlossene Stellung zu bringen ist, wobei einander zugewandte Seiten, insbesondere Innenseiten, der Instrumentenbranchen jeweils als Greifflächen zum klemmenden Halten von zu koagulierendem Gewebe in der geschlossenen Stellung ausgebildet sind und jeweils zumindest eine Elektrodenfläche aufweisen.

Eine leistungsstarke Alternative zur Gewebeverbindung besteht darin, Gewebe mit HF-Strom (Hochfrequenz-Strom) zu koagulieren, beispielsweise indem das Gewebe zwischen zwei HF-Elektroden mit einem HF-Strom beaufschlagt wird. Durch die Beaufschlagung mit HF-Strom kommt es mindestens teilweise zu einer Denaturierung von Gewebsproteinen, wobei beispielsweise tripelhelikales Kollagen, ein Hauptbestandteil von Bindegewebe, in kollagene Einzelhelices zerlegt wird. Die denaturierten Proteinbestandteile lassen sich dabei ohne Weiteres unter Erzielung einer Gewebeverbindung miteinander fusionieren.

Zum Verschweißen von Gewebe wird zwischen zwei Klemmbacken oder Instrumentenbranchen gefasstes Gewebe mit Strom beaufschlagt, der zwischen Elektroden der Backen/Branchen fließt. Damit es nicht zum Versagen der Versiegelung bzw. Verschweißung kommt, müssen auf das Gewebe einwirkende und beim Verschweißen vorliegende Parameter erfasst und geregelt werden. Um dies zu gewährleisten, benötigt man eine genaue Kontrolle von Temperatur, Druck, Gewebeimpedanz sowie Abstand und Lage der Elektroden.

Es ist wünschenswert, zwischen den Klemmbacken gehaltenes Gewebe gleichmäßig zu behandeln, so dass alle Bereiche zuverlässig erreicht werden und keiner mit zu hohen Strom beaufschlagt wird. Dazu muss sichergestellt werden, dass die HF-Elektroden gleichmäßig voneinander beabstandet sind bzw. parallel zueinander ausgerichtet sind.

Aus dem Stand der Technik sind Koagulationsinstrumenten kleinerer Bauart bekannt, wie z.B. in EP 1 747 762 A2 gezeigt, bei denen es bedingt durch die Bauart beim Schließen der Klemmbacken zu einer nicht parallelen Ausrichtung der HF-Elektroden kommen kann, beispielsweise infolge von Durchbiegung. Dies führt zu einer Reduzierung des Abstands zwischen den Elektroden, im ungünstigsten Fall können Kurzschlüsse entstehen.

Es ist bekannt, den Abstand zwischen Elektroden eines Koagulationsinstruments durch zwischen den Elektroden angebrachte Abstandshalter einzuhalten. Wenn jedoch auf den Klemmbacken eine größere Anzahl von Abstandshaltern vorgesehen ist, wie dies z.B. in EP 1 656 901 B1, EP1 952 777 A1, EP 1 372 507 A1 oder US 2004/122423 A1, gezeigt ist, perforieren die Abstandshalter zwangsläufig das zu behandelnde Gewebe, da das Gewebe unter den Abstandhaltern bei geschlossenen Klemmbacken derart komprimiert wird, dass es zu dauerhaften Gewebeschädigungen kommt. Dies hat negative Auswirkungen auf das Ergebnis der Versiegelung.

Die Druckschriften US 2003/0216733 A1 und EP 2 491 880 A1 offenbaren jeweils eine Vorrichtung zur Gewebebehandlung mit einem Maulteil, an dessen Klemmbacken die Elektroden relativ zu den Greifflächen oder Isolierstegen versenkt sind.

Da die Abstandshalter ferner aus elektrisch nicht leitendem Material sind, um einen Kurzschluss zwischen den HF-Elektroden zu vermeiden, entsteht im Bereich dieser Abstandshalter ein sog. Koagulationsschatten, d.h. dass die Gewebeabschnitte im Bereich der oder unter den Abstandshaltern abgekapselt sind, somit nicht oder nur ungenügend mit Strom beaufschlagt werden und es dort zu keiner zufriedenstellenden Verschweißung der Gefäßabschnitte kommt. Außerdem hat es sich gezeigt, dass derartige, elektrisch nichtleitende Abstandshalter insbesondere dann, wenn sie auf der Elektrode beispielsweise durch Kleben befestigt werden, leicht abplatzen können, und dann ggf. unbemerkt in den Patientenkörper gelangen. Darüber hinaus ist in einem solchen Fall der vordefinierte Elektrodenabstand nicht mehr gewährleistet.

Zusammenfassend kann festgestellt werden, dass bei bekannten Instrumenten zur HF-Versiegelung von Gewebe unter Umständen Kurzschlüsse auftreten können, was das Ergebnis der Koagulation in der Regel negativ beeinflusst und darüber hinaus Gewebeanhaftungen an den Elektroden begünstigen kann. Abstandhalter zwischen den Elektroden bzw. den Elektrodenflächen verschlechtern das Ergebnis der Koagulation ebenfalls. Insbesondere an Elektrodenecken kann es zu Stromkonzentrationen kommen, die das Koagulationsergebnis ebenfalls negativ beeinflussen können. Nicht elektrisch isolierte Elektroden verursachen Kolateralschäden am Gewebe.

Vor diesem Hintergrund liegt der vorliegenden Erfindung die Aufgabe zugrunde, ein Instrument bereitzustellen, das eine reproduzierbare Versiegelung von Gewebe, insbesondere von Humangewebe, mittels Thermofusions-Technik ermöglicht, wobei elektrische Kurzschlüsse der Elektroden mit hoher Sicherheit vermieden werden und Gewebeanhaftungen an den Elektroden reduziert bzw. vermieden werden können. Des Weiteren sollen Kolateralschäden am Gewebe durch elektrisch nicht leitende Maulteile wie separate Abstandshalter, vermieden bzw. reduziert werden. Das Instrument soll allgemein bei Gewebeverbindungen, unter anderem zur End-zu-End-Anastomose von Hohlgefäßen wie Dünn- und Dickdarm, geeignet sein.

Diese Aufgabe wird nach der vorliegenden Erfindung gelöst durch ein elektrochirurgisches Instrument, insbesondere bipolares Instrument, gemäß Anspruch 1. Insbesondere ist zumindest eine der Elektrodenflächen innerhalb der an derselben Instrumentenbranche befindlichen Greiffläche angeordnet und relativ zu dieser versenkt. Die Greifflächen bzw. eine diese aufweisende oder ausbildende Struktur besteht insbesondere aus einem elektrisch isolierenden Material.

Man kann auch sagen, dass durch die Erfindung die Elektrodenfläche gegenüber der Greiffläche der entsprechenden Instrumentenbranche zurückversetzt angeordnet oder ausgebildet ist. Sie ist in eine von der gegenüberliegenden Instrumentenbranche abgewandte Richtung versetzt. Im Ergebnis ist im geschlossenen Zustand der beiden Branchen jede Elektrodenfläche der einen Instrumentenbranche von jeder Elektrodenfläche der anderen Instrumentenbranche beabstandet, während die Greifflächen vorspringen und die gegenseitigen Kontaktmöglichkeiten der Branchen ausbilden. Zumindest eine der Greifflächen bildet so einen Abstandhalter zwischen den Instrumentenbranchen, so dass ein Kurzschluss zwischen den Elektroden der Branchen nicht möglich ist. Dieser Abstandhalter ist in vorteilhafter Weise außerhalb der Elektrodenfläche angeordnet, so dass die Koagulation nicht negativ beeinflusst wird. Anders als bei bekannten Instrumenten, bei denen zu koagulierendes Gewebe mittels der Elektrodenflächen, die mittels separat vorgesehenen isolierenden Abstandhaltern auf Distanz gehalten werden, gehalten und geklemmt wird, halten und klemmen bei einem erfindungsgemäßen Instrument die elektrisch isolierenden Greifflächen zu koagulierendes Gewebe. Die Greifflächen bzw. die diese ausbildende Struktur können in vorteilhafter Weise die tragenden Elemente der Branchen ausbilden oder sind von diesen getragen und übernehmen vom Gewebe beim Einklemmen von Gewebe eingeleiteten Kräfte und Momente. Die Elektrodenflächen ausbildende Elektroden bleiben bei der Erfindung von solchen Kräften und Momenten unbelastet, so dass deren Verformungen sicher und einfach verhindert oder zumindest vermindert werden können. Im Ergebnis können die Elektroden, die in der Regel aus einem teuren Werkstoff bestehen, geringer dimensioniert werden als bei Instrumenten des Standes der Technik. Die tragende und Kräfte und Momente aufnehmende Struktur kann bzw. die Greifflächen können in der jeweils erforderlichen Dimensionierung aus günstigem Material bestehen, wie zum Beispiel (thermoplastischen) Kunststoff oder Keramik.

Bei Elektrodenflächen, welche lediglich lokal über nicht leitende Abstandshalter von einander beabstandet oder getrennt werden, wie sie aus dem Stand der Technik bekannt sind, besteht oftmals die Gefahr, dass sich die Instrumentenbranchen bei entsprechend großer Klemmkraft zwischen den Abstandshaltern durchbiegen und es zu einem Kurzschluss zwischen den gegenüberliegenden Elektroden kommt. Bei der erfindungsgemäßen Konfiguration dagegen können sich selbst dann, wenn die beiden Instrumentenbranchen bei hoher Klemmkraft mit ihren elektrisch nicht leitenden Greifflächen plan an einander liegen, die gegenüberliegenden Elektrodenflächen nicht kontaktieren, da diese zumindest an einer Instrumentenbranche in der Greiffläche versenkt sind. Dadurch wird auch eine bessere Planparallelität der Greifflächen und somit auch der gegenüberliegenden Elektrodenflächen erzielt werden.

Vorteilhafte Ausführungsformen der Erfindung sind in den Unteransprüchen beansprucht und werden nachfolgend näher erläutert.

Nach einer Ausführungsform der Erfindung beträgt in der geschlossenen Stellung der Abstand zwischen einander gegenüberliegenden Elektrodenflächen beider Instrumentenbranchen zwischen 20µm und 200µm, vorzugsweise zwischen 40µm und 170µm, bevorzugter zwischen 80µm und 150µm und besonders bevorzugt zwischen 95µm und 110µm. Alternativ oder zusätzlich kann der Abstand zwischen der Elektrodenfläche einer Instrumentenbranche und der Greiffläche dieser Instrumentenbranche zwischen 10µm und 100µm, vorzugsweise zwischen 10µm und 80µm, bevorzugter zwischen 10µm und 65µm und besonders bevorzugt zwischen 10µm und 50µm betragen.

Eine besonders einfache Handhabung, bei der die Orientierung des Instruments im Koagulationsbereich gleichgültig ist, kann erreicht werden, wenn die Elektrodenflächen beider Instrumentenbranchen jeweils gleichweit von der Greiffläche der jeweiligen Instrumentenbranche zurückversetzt angeordnet sind. Es ist jedoch auch im Rahmen der Erfindung, wenn die Elektrodenflächen unterschiedlich weit von der jeweiligen Greiffläche beabstandet sind. Alternativ kann zumindest eine Elektrodenfläche einer Instrumentenbranche mit deren Greiffläche fluchten, vorzugsweise eben fluchten, d.h. dass die Elektrodenflächen und Greiffläche einer Instrumentenbranche bündig abschließen bzw. in einer Ebene liegen. In diesem Fall ist die entsprechende gegenüberliegende Elektrodenfläche bzw. sind die entsprechenden gegenüberliegenden Elektrodenflächen gegenüber der Greiffläche ihrer Instrumentenbranche nach hinten versetzt angeordnet, so dass kein Kurzschluss auftreten kann.

Nach einer besonders vorteilhaften Ausführungsform ist zumindest ein Teilbereich einer Greiffläche mit einer Oberflächenprofilierung oder einer Oberflächenstruktur versehen. Nach einer Weiterbildung sind zumindest einander gegenüberliegende Bereiche, zumindest Teilbereiche, der Greifflächen beider Instrumentenbranchen derart ausgebildet. Es kann von Vorteil sein, wenn eine der Greifflächen, vorzugsweise beide Greifflächen der beiden Instrumentenbranchen, vollständig mit einer solchen Profilierung oder Struktur versehen sind. Durch Vorsehen einer derartigen Oberflächenprofilierung/Oberflächenstruktur kann ein sicheres Klemmen oder Halten von Gewebe zwischen den in der geschlossenen Stellung befindlichen Branchen bewirkt werden, ohne dass dazu ein hoher Klemmdruck erforderlich ist, was besonders gewebeschonend ist und Kolateralschäden im Gewebe zu vermeiden hilft.

Kurzschlussvermeidung und Klemmen/Halten von Gewebe kann nach einer weiteren Form der Erfindung verbessert werden, indem eine der Elektrodenflächen, vorzugsweise jede Elektrodenfläche, zumindest einer Instrumentenbranche, vorzugsweise beider Branchen, vollumfänglich, zumindest auf beiden Längseiten, wobei die Kurzseiten frei sind, von der Greiffläche der dazugehörigen Instrumentenbranche umgeben ist. So kann sichergestellt sein, dass bei leichter Verformung der Instrumentenbranchen gegenseitiger Kontakt der Elektrodenflächen und damit Kurzschlüsse nicht auftreten. Insbesondere können die Greifflächen mit einem auf den einander zugewandte Seiten der Instrumentenbranchen umlaufenden Rand, vorzugsweise ununterbrochen umlaufenden Rand, ausgebildet sein.

Eine weitere Verbesserung einer sicheren und reproduzierbaren Beabstandung der Elektrodenflächen voneinander sowie der Stabilität der Branchen wird erfindungsgemäß erreicht, indem die Greifflächen in Form einer netzartigen Matrix mit isolierenden Greifflächenstegen und zwischen diesen liegenden und die Elektrodenflächen freigebenden Lücken ausgebildet sind.

Gemäß einem Aspekt der Erfindung können die Elektrodenflächen können von einer Vielzahl an unabhängigen und gegebenenfalls unabhängig ansteuerbaren Elektroden oder von einer einzigen zusammenhängenden Elektrode stammen, deren Elektrodenflächen von an vorbestimmten Stellen vorgesehenen Ausnehmungen in der Greiffläche freigegeben wird.

Nach einer Ausführungsform der Erfindung ist die Greiffläche an einer oder durch eine Stützstruktur ausgebildet, insbesondere einteilig. Vorzugsweise besteht diese aus einem elektrisch isolierenden Material. Auf diese Weise lässt sich die Greiffläche besonders einfach fertigen, da nicht mehr einzelne Abstandshalter vorgesehen oder auf den Elektroden aufgebracht werden müssen. Bei einer Weiterbildung kann zumindest eine der Instrumentenbranchen ein elektrisch isolierendes Außengehäuse aufweisen, das einteilig mit der Greiffläche der Instrumentenbranche ausgebildet ist. Es ist insbesondere im Rahmen der Erfindung, dass eine vorstehend genannte Stützstruktur als solches Gehäuse ausgebildet ist. Die Stützstruktur oder das Gehäuse umgeben die jeweilige die Elektrodenfläche(n) ausbildende Elektrode mit Ausnahme der Elektrodenfläche(n) vollständig, d.h. schirmen sie gegenüber der Umgebung in alle übrigen Raumrichtungen isolierend ab.

Ein Instrument nach der Erfindung kann besonders einfach und günstig hergestellt werden und haltbar und robust ausgebildet sein, indem die Elektrodenflächen durch eine mittels 2K-CIM-Technologie in das Gehäuse eingebetteten Elektrode ausgebildet sind, wobei der nichtleitende Teil, das Gehäuse, aus einer Keramik, z.B. Al₂O₃, besteht und die Elektrode aus einer elektrisch leitenden Mischkeramik, z.B. Al₂O₃ + TiN besteht.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 einen Abschnitt eines erfindungsgemäßen Instruments in einer schematischen seitlichen Ansicht,
Fig. 2 eine Aufsicht auf eine Klemmseite eines Instrumentenmaulteils des Instruments der Fig. 1,
Fig. 3 eine seitliche Ansicht des Instrumentenmaulteils der Fig. 2, und
Fig. 4 eine Schnittansicht entlang der Linie 4-4 in Fig. 2.

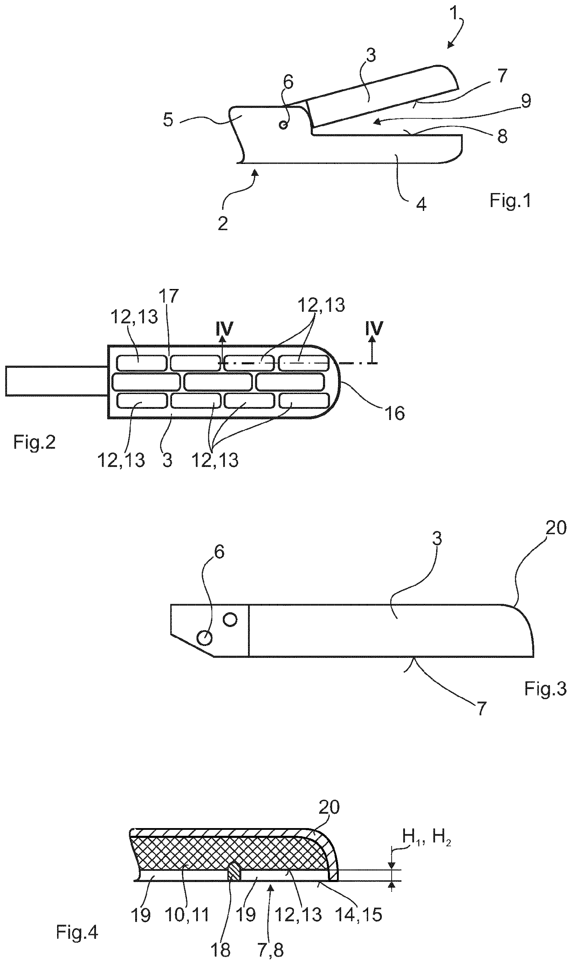

Die Figuren 1 bis 4 zeigen ein Maulteil 2 eines Instruments 1 nach der Erfindung. Das Maulteil 2 weist eine erste Instrumentenbranche 3 und eine zweite Instrumentenbranche 4 auf. Die erste Instrumentenbranche 3 ist einteilig mit einem in Fig. 1 angedeuteten Instrumentenkörper 5 ausgebildet bzw. nicht relativbeweglich zu dieser daran angeordnet. Die zweite Instrumentenbranche 4 ist mittels eines Gelenks 6 oder Scharniers 6 an dem Instrumentenkörper 5 oder an der ersten Instrumentenbranche 3 angelenkt. Die zweite Instrumentenbranche 4 ist daher um das Gelenk 6 relativ zur ersten Instrumentenbranche 3 zwischen einer in Fig. 1 gezeigten geöffneten Stellung und einer in den Figuren nicht gezeigten geschlossenen Stellung, in der die Instrumentenbranchen 3, 4 flächig aneinander anliegen, schwenkbar.

Beide Instrumentenbranchen 3, 4 weisen einander zugewandte Innenseiten 7 bzw. 8 auf. Zwischen diesen und damit zwischen den Instrumentenbranchen 3, 4 ist ein Zwischenraum 9 ausgebildet, in dem mittels des Instruments 1 zu koagulierendes Gewebe eingeführt wird.

Die erste Instrumentenbranche 3 weist eine erste Elektrode 10 und die zweite Instrumentenbranche 4 eine zweite Elektrode 11 auf. Auf der dem Zwischenraum 9 zugewandten Seite (Innenseiten 7, 8) weist die erste Elektrode 10 eine erste Elektrodenfläche 12 und die zweite Elektrode 11 eine zweite Elektrodenfläche 13 auf. Die erste und die zweite Elektrode 10, 11 sind mittels einer in den Figuren nicht gezeigten und im Instrumentenkörper 5 aufgenommenen elektrischen Schaltanordnung mit einem HF-Strom zu beschalten. Zum Koagulieren von Gewebe werden die beiden Instrumentenbranchen 3, 4 aus der in Figur 1 gezeigten geöffneten Stellung in eine in den Figuren nicht dargestellte geschlossene Stellung überführt und mit einem HF-Strom beaufschlagt.

Auf der dem Zwischenraum 9 und damit der gegenüberliegenden Instrumentenbranche 4 zugewandten Seite ist an der Instrumentenbranche 3 eine erste Greiffläche 14 ausgebildet. In entsprechender Weise ist auf der dem Zwischenraum 9 und damit der gegenüberliegenden Instrumentenbranche 3 zugewandten Seite an der Instrumentenbranche 4 eine zweite Greiffläche 15 ausgebildet. Wie insbesondere aus einer Zusammenschau der Figuren 2 und 4 zu erkennen ist, ist die erste Greiffläche 13 aus zahlreichen Teilgreifflächen oder Greifflächenabschnitten gebildet. Sie weist zum einen außen umlaufenden Greifflächenrandabschnitt 16 und zum anderen einen innerhalb des Greifflächenrandabschnitts 16 ausgebildete Greifflächenmatrix 17 auf. Diese besteht im Wesentlichen aus Greifflächenstegen 18, zwischen denen Zwischenräume 19 ausgebildet sind, die die Elektrodenflächen 12 freigeben und nicht abdecken. Die Greifflächen 14, 15 bilden Kontaktbereiche aus, mit denen die Branchen 3, 4 Gewebe bzw. gegebenenfalls die gegenüberliegende Branche 3, 4 berühren. Die Greifflächen 14, 15 sind elektrisch isolierend.

Figur 4 zeigt, dass die erste Elektrodenfläche 12 innerhalb der ersten Greiffläche 14 angeordnet ist und relativ zu dieser versenkt ist. Die erste Elektrodenfläche 12 ist so gegenüber der ersten Greiffläche 14 der ersten Instrumentenbranche 3 zurückversetzt angeordnet oder ausgebildet. Sie ist in eine von der gegenüberliegenden zweiten Instrumentenbranche 4 abgewandte Richtung versetzt. Der Versatz der ersten Elektrodenfläche 12 gegenüber der ersten Greiffläche 14 ist in Figur 4 mittels der Teilspalthöhe H₁ gekennzeichnet. Die zweite Elektrodenfläche 13 und die zweite Greiffläche 15 der zweiten Instrumentenbranche 4 können in entsprechender Weise ausgebildet sein. Der Versatz der zweiten Elektrodenfläche 13 gegenüber der zweiten Greiffläche 15 ist in Figur 4 mittels der Teilspalthöhe H₂ gekennzeichnet. Bei geschlossenem Maulteil 1 summieren sich die Teilspalthöhen H₁ und H₂ zur Spalthöhe H zwischen den Elektrodenflächen 12, 13.

Fig. 4 zeigt des Weiteren, dass die erste Greiffläche 14 auf der der zweiten Instrumentenbranche 4 zugewandten Seite einer aus einem elektrisch isolierenden Material bestehenden Gehäusestruktur 20 ausgebildet ist. Die erste Elektrode 10 ist innerhalb der Gehäusestruktur 20 in der vorstehend beschriebenen Weise aufgenommen, insbesondere mittels eines 2K-Verfahrens in die Gehäusestruktur 20 eingegossen oder eingespritzt. Die Gehäusestruktur 20 bildet zum einen eine die Elektroden 10, 11 jeweils umgebende elektrische Isolierung und zum anderen einen Abstandhalter aus, der ein Inkontaktgelangen der beiden Elektroden 10, 11 verhindert. Durch die erfindungsgemäße Ausgestaltung können die Elektroden 10, 11 in oder an einer tragenden Struktur angeordnet oder von dieser gehalten sein, die vom zu koagulierenden Gewebe eingeleitete Kräfte und Momente übernimmt und in den Instrumentenkörper 5 ableitet, ohne dass derartige Kräfte und Momente eine Verformung der Elektroden 10, 11 hervorrufen könnten.

### Bezugszeichenliste

- 1: HF-Instrument
- 2: Maulteil
- 3: erste Instrumentenbranche
- 4: zweite Instrumentenbranche
- 5: Instrumentenkörper
- 6: Gelenk, Scharnier
- 7: Innenseite
- 8: Innenseite
- 9: Zwischenraum
- 10: erste Elektrode
- 11: zweite Elektrode
- 12: erste Elektrodenfläche
- 13: zweite Elektrodenfläche
- 14: erste Greiffläche
- 15: zweite Greiffläche
- 16: Greifflächenrandabschnitt
- 17: Greifflächenmatrix
- 18: Greifflächensteg
- 19: Zwischenraum
- 20: Gehäusestruktur

## Patentansprüche

1. Elektrochirurgisches Instrument (1) zur Elektrokoagulation von Gewebe, mit einem Maulteil (2), welches zwei Instrumentenbranchen (3, 4) aufweist, von denen zumindest eine gegenüber der anderen bewegbar ist, so dass das Maulteil (2) in eine geöffnete Stellung oder in eine geschlossene Stellung bringbar ist, wobei einander zugewandte Seiten (7, 8) der Instrumentenbranchen (3, 4) jeweils als Greifflächen (14, 15) zum klemmenden Halten von zu koagulierendem Gewebe in der geschlossenen Stellung ausgebildet sind und jeweils zumindest eine Elektrodenfläche (12, 13) aufweisen, wobei
die Greifflächen (14, 15) elektrisch isolierend ausgebildet sind;
die zumindest eine Elektrodenfläche (12, 13) in der Greiffläche (14, 15) der jeweiligen Instrumentenbranche (3, 4) angeordnet ist; und
an zumindest einer Instrumentenbranche (3, 4) die zumindest eine Elektrodenfläche (12, 13) relativ zu deren Greiffläche (14, 15) versenkt ist;
**dadurch gekennzeichnet, dass**
die Greifflächen (14, 15) in Form einer netzartigen Matrix (17) mit isolierenden Greifflächenstegen (18) und zwischen diesen liegenden und die Elektrodenflächen (12, 13) freigebenden Lücken (19) ausgebildet sind.

2. Elektrochirurgisches Instrument (1) nach Anspruch 1, **dadurch gekennzeichnet, dass** der Abstand zwischen einander gegenüberliegenden Elektrodenflächen (12, 13) beider Instrumentenbranchen (3, 4) zwischen 20µm und 200µm, vorzugsweise zwischen 40µm und 170µm, bevorzugter zwischen 80µm und 150µm und besonders bevorzugt zwischen 95µm und 110µm beträgt und/oder dass der Abstand zwischen der Elektrodenfläche (12, 13) einer Instrumentenbranche (3, 4) und der Greiffläche (14, 15) dieser Instrumentenbranche zwischen 10µm und 100µm, vorzugsweise zwischen 10µm und 80µm, bevorzugter zwischen 10µm und 65µm und besonders bevorzugt zwischen 10µm und 50µm beträgt.

3. Elektrochirurgisches Instrument (1) nach Anspruch 1 oder 2, **dadurch gekennzeichnet, dass** die Elektrodenflächen (12, 13) beider Instrumentenbranchen (3, 4) jeweils gleichweit von der Greiffläche (14, 15) der jeweiligen Instrumentenbranche (3, 4) zurückversetzt angeordnet sind.

4. Elektrochirurgisches Instrument (1) nach Anspruch 1 oder 2, **dadurch gekennzeichnet, dass** zumindest eine Elektrodenfläche (12, 13) einer Instrumentenbranche (3, 4) mit deren Greiffläche (14, 15) fluchtet, vorzugsweise eben fluchtet.

5. Elektrochirurgisches Instrument (1) nach einem der vorstehenden Ansprüche, **dadurch gekennzeichnet, dass** zumindest ein Teilbereich einer Greiffläche (14, 15) mit einer Oberflächenprofilierung oder einer Oberflächenstruktur versehen ist.

6. Elektrochirurgisches Instrument (1) nach einem der vorstehenden Ansprüche, **dadurch gekennzeichnet, dass** eine Elektrodenfläche (12, 13) zumindest einer Instrumentenbranche (3, 4), vorzugsweise alle Elektrodenflächen (12, 13), vollumfänglich von der Greiffläche (14, 15) der dazugehörigen Instrumentenbranche (3, 4) umgeben ist.

7. Elektrochirurgisches Instrument (1) nach einem der vorhergehenden Ansprüche, **dadurch gekennzeichnet, dass** zumindest eine der Instrumentenbranchen (3, 4) ein elektrisch isolierendes Außengehäuse (20) aufweist, das einteilig mit der Greiffläche (14, 15) der Instrumentenbranche (3, 4) ausgebildet ist, insbesondere aus Kunststoff und/oder Keramik.

8. Elektrochirurgisches Instrument (1) nach einem der vorhergehenden Ansprüche, **dadurch gekennzeichnet, dass** die Greifflächen (14, 15) mit einem auf den einander zugewandten Seiten der Instrumentenbranchen (3, 4) umlaufenden Greifflächenrandabschnitt (16), vorzugsweise ununterbrochen umlaufend, ausgebildet sind.

9. Elektrochirurgisches Instrument (1) nach einem der vorhergehenden Ansprüche, **dadurch gekennzeichnet, dass** die Elektrodenflächen (12, 13) durch eine mittels 2K-CIM-Technologie in das Gehäuse eingebetteten Elektrode (10, 11) ausgebildet sind, insbesondere aus einer elektrisch leitenden Mischkeramik bestehen.

## Claims

1. An electrosurgical instrument (1) for the electrocoagulation of tissue, comprising a jaw part (2) which has two instrument branches (3, 4) of which at least one is movable in relation to the other so that the jaw part (2) can be brought into an opened position or into a closed position, wherein facing sides (7, 8) of the instrument branches (3, 4) are respectively configured as grip faces (14, 15) for the clamping hold of tissue to be coagulated in the closed position and have at least one electrode face (12, 13) each, wherein
the grip faces (14, 15) are configured to be electrically insulating;
the at least one electrode face (12, 13) is arranged in the grip face (14, 15) of the respective instrument branch (3, 4); and
the at least one electrode face (12, 13) is sunk in relative to the grip face (14, 15) thereof on at least one instrument branch (3, 4);
**characterized in that**
the grip faces (14, 15) are in the form of a net-like matrix (17) having insulating grip face lands (18) and clearances (19) located therebetween and exposing the electrode faces (12, 13).

2. The electrosurgical instrument (1) according to claim 1, **characterized in that** the distance between opposing electrode faces (12, 13) of both instrument branches (3, 4) ranges from 20µm to 200µm, preferably from 40µm to 170µm, more preferred from 80µm to 150µm and especially preferred from 95µm to 110µm, and/or that the distance between the electrode face (12, 13) of one instrument branch (3, 4) and the grip face (14, 15) of said instrument branch ranges from 10µm to 100µm, preferably from 10µm to 80µm, more preferred from 10µm to 65µm and especially preferred from 10µm to 50µm.

3. The electrosurgical instrument (1) according to claim 1 or 2, **characterized in that** the electrode faces (12, 13) of both instrument branches (3, 4) are arranged to be equally recessed from the grip face (14, 15) of the respective instrument branch (3, 4).

4. The electrosurgical instrument (1) according to claim 1 or 2, **characterized in that** at least one electrode face (12, 13) of one instrument branch (3, 4) is aligned, preferably flatly aligned, with the grip face (14, 15) thereof.

5. The electrosurgical instrument (1) according to any one of the preceding claims, **characterized in that** at least one segment of a grip face (14, 15) is provided with a surface profile or a surface structure.

6. The electrosurgical instrument (1) according to any one of the preceding claims, **characterized in that** one electrode face (12, 13) of at least one instrument branch (3, 4), preferably all electrode faces (12, 13), is/are fully surrounded by the grip face (14, 15) of the dedicated instrument branch (3, 4).

7. The electrosurgical instrument (1) according to any one of the preceding claims, **characterized in that** at least one of the instrument branches (3, 4) includes an electrically insulating outer housing (20) which is formed in one piece with the grip face (14, 15) of the instrument branch (3, 4), especially made from plastic and/or ceramic.

8. The electrosurgical instrument (1) according to any one of the preceding claims, **characterized in that** the grip faces (14, 15) are configured to have a grip face rim portion (16) which is peripheral, preferably continuously peripheral, on the facing sides of the instrument branches (3, 4).

9. The electrosurgical instrument (1) according to any one of the preceding claims, **characterized in that** the electrode faces (12, 13) are configured by an electrode (10, 11) embedded in the housing by means of 2K-CIM technology and are especially made from electrically conductive mixed ceramics.

## Revendications

1. Instrument électrochirurgical (1) pour l'électrocoagulation de tissu, avec une partie mâchoire (2), laquelle présente deux branches d'instrument (3, 4), dont au moins l'une est mobile par rapport à l'autre, de sorte que la partie mâchoire (2) peut être amenée dans une position ouverte ou dans une position fermée, dans lequel les faces (7, 8) tournées l'une vers l'autre des branches d'instrument (3, 4) sont réalisées respectivement sous la forme de surfaces de préhension (14, 15) destinées à retenir par serrage du tissu à coaguler dans la position fermée et présentent respectivement au moins une surface d'électrode (12, 13), dans lequel
les surfaces de préhension (14, 15) sont réalisées de manière électriquement isolante ;
l'au moins une surface d'électrode (12, 13) est agencée dans la surface de préhension (14, 15) de la branche d'instrument (3, 4) respective ; et
l'au moins une surface d'électrode (12, 13) est enfoncée sur au moins une branche d'instrument (3, 4) par rapport à la surface de préhension (14, 15) de celle-ci ;
**caractérisé en ce que**
les surfaces de préhension (14, 15) sont réalisées sous la forme d'une matrice (17) du type filet avec des éléments jointifs de surface de préhension (18) isolants et des intervalles (19) situés entre ceux-ci et libérant les surfaces d'électrode (12, 13).

2. Instrument électrochirurgical (1) selon la revendication 1, **caractérisé en ce que** l'écart entre des surfaces d'électrode (12, 13) opposées l'une à l'autre des deux branches d'instrument (3, 4) est compris entre 20 µm et 200 µm, de préférence entre 40 µm et 170 µm, de manière plus préférée entre 80 µm et 150 µm et de manière de loin préférée entre 95 µm et 110 µm et/ou **en ce que** l'écart entre la surface d'électrode (12, 13) d'une branche d'instrument (3, 4) et la surface de préhension (14, 15) de cette branche d'instrument est compris entre 10 µm et 100 µm, de préférence entre 10 µm et 80 µm, de manière plus préférée entre 10 µm et 65 µm et de manière de loin préférée entre 10 µm et 50 µm.

3. Instrument électrochirurgical (1) selon la revendication 1 ou 2, **caractérisé en ce que** les surfaces d'électrode (12, 13) des deux branches d'instrument (3, 4) sont agencées respectivement de manière décalée à équidistance de la surface de préhension (14, 15) de la branche d'instrument (3, 4) respective.

4. Instrument électrochirurgical (1) selon la revendication 1 ou 2, **caractérisé en ce qu'**au moins une surface d'électrode (12, 13) d'une branche d'instrument (3, 4) est en affleurement avec la surface de préhension (14, 15) de celle-ci, de préférence est en affleurement plan.

5. Instrument électrochirurgical (1) selon l'une quelconque des revendications précédentes, **caractérisé en ce qu'**au moins une zone partielle d'une surface de préhension (14, 15) est pourvue d'un profilage de surface ou d'une structure de surface.

6. Instrument électrochirurgical (1) selon l'une quelconque des revendications précédentes, **caractérisé en ce qu'**une surface d'électrode (12, 13) d'au moins une branche d'instrument (3, 4), de préférence toutes les surfaces d'électrode (12, 13), est entièrement entourée par la surface de préhension (14, 15) de la branche d'instrument (3, 4) associée.

7. Instrument électrochirurgical (1) selon l'une quelconque des revendications précédentes, **caractérisé en ce qu'**au moins une des branches d'instrument (3, 4) présente un boîtier extérieur (20) électriquement isolant, qui est réalisé d'une seule pièce avec la surface de préhension (14, 15) de la branche d'instrument (3, 4), en particulier en plastique et/ou en céramique.

8. Instrument électrochirurgical (1) selon l'une quelconque des revendications précédentes, **caractérisé en ce que** les surfaces de préhension (14, 15) sont réalisées, de préférence en s'étendant de manière ininterrompue, avec une portion bord de surface de préhension (16) s'étendant sur les faces tournées l'une vers l'autre des branches d'instrument (3, 4).

9. Instrument électrochirurgical (1) selon l'une quelconque des revendications précédentes, **caractérisé en ce que** les surfaces d'électrode (12, 13) sont réalisées par une électrode (10, 11) incorporée dans le boîtier par technologie 2K-CIM, en particulier sont constituées d'une céramique mixte électroconductrice.
